# EUROPÄISCHE PATENTSCHRIFT

(11) **EP 2 111 238 B1**
(45) Veröffentlichungstag und Bekanntmachung des Hinweises auf die Patenterteilung: **31.07.2013**
(21) Anmeldenummer: 07856958.9
(22) Anmeldetag: 20.12.2007
(51) Int. Cl.: A61L 2/20, H05B 6/10, B65B 55/10

(54) **VERFAHREN UND VORRICHTUNG ZUR STERILISATION VON BEHÄLTERN AUS METALL**
METHOD AND DEVICE FOR STERILISING METAL CONTAINERS
PROCÉDÉ ET DISPOSITIF DE STÉRILISATION DE CONTENANTS MÉTALLIQUES

(30) Priorität: 13.01.2007 DE 102007001970
(43) Veröffentlichungstag der Anmeldung: 28.10.2009
(73) Patentinhaber: KHS GmbH, 44143 Dortmund (DE)
(72) Erfinder: LOTHAR, Wilhelm, 61184 Karben (DE)
(86) Internationale Anmeldenummer: PCT/EP2007/011235
(87) Internationale Veröffentlichungsnummer: WO 2008/083824

(56) Entgegenhaltungen:
- EP-A- 1 607 106
- WO-A-99/30747
- JP-A- 11 278 443
- JP-A- 2005 170 393
- US-A- 3 961 150
- US-A- 4 742 667
- US-A1- 2003 165 400
- US-A1- 2003 230 567

## Beschreibung

Die Erfindung betrifft ein Verfahren und eine Vorrichtung zur Sterilisation von Behältern aus Metall, insbesondere Dosen.

Die Sterilisation von Verpackungsbehältern, beispielsweise in der Lebensmittel- oder Getränkeindustrie, gewinnt zunehmend an Bedeutung, da eine lange Lagerfähigkeit des Behälterinhaltes ohne die Zugabe von Konservierungsstoffen oder eine anschließende Behandlung, beispielsweise durch pasteurisieren, aufgrund ihrer negativen Einwirkung auf das verpackte Produkt zunehmend unattraktiver werden.

Um dies zu vermeiden kommen z.B. bei der Befüllung von Flaschen sogenannte aseptische Füllanlagen zum Einsatz. Hierzu wird die gesamte Abfüllanlage In einem sterilen Umfeld betrieben. Dazu wird die Anlage eingehaust, das Innere sterilisiert und möglichst keimfrei gehalten, sowie dafür gesorgt, dass die in die Anlage geleitete Luft und alle sonstigen Substanzen Im Inneren praktisch keimfrei sind. Ein wesentlicher Baustein ist außerdem, dass das Innere der zu befüllenden Behälter ebenfalls sterilisiert wird. Aufgrund unterschiedlicher Probleme, die bei der Verwendung von metallischen Behältern auftreten, wurden derartige Anlagen für den Gebrauch mit metallischen, zu befüllenden Behältern, wie beispielsweise Getränkedosen, noch nicht zufriedenstellend realisiert.

Bekannt sind die US 4 742 667 A und JP 2005 170393 A offenbaren ein sehr bekanntes Verfahren zu Sterilisierung von Behältern, bei welchem H₂O₂ als Dampf oder Spray auf den Oberflächen der Behälter aufgebracht wird und anschließend wird das H₂O₂ aktiviert, indem heize Luft auf bzw. über die so benetzten Oberflächen geleitet wird. Eine Weiterentwicklung zeigt die EP 1 607 106 A, bei welcher mittels Katalysator, der an der inneren Oberfläche einer Verdampfereinheit angeordnet ist, die Aktivierung des H₂O₂ eingeleitet wird.

US 3 961 150 A zeigt einen so genannten Pasteur und ein Verfahren hierzu, bei welchem der verschlossene und gefüllte Behälter elektrisch erwärmt wird, ähnlich wie bei einem Autoklavieren. Eine Variante desselben technologischen Konzeptes zeigt die JP 11 278443 A, bei welcher eine Erhitzung der verschlossenen Behälter mittel induktiver Erwärmung erfolgt. Hier besteht der Nachteil darin, dass Lebensmittil zum Teil durch Wärmeeinwirkung negativ beeinflusst werden.

Zur Sterilisation derartiger Behälter steht eine Reihe von Verfahren zur Verfügung. Behälter aus Metall werden gegenwärtig, wenn überhaupt, durch Dampfsterilisation oder im Batchprozess sterilisiert. Ansonsten finden sie für die beschriebene aseptische Abfüllung noch keine Anwendung.

Weitere bekannte Sterilisationsverfahren benutzen unterschiedliche chemische Sterilisationsmedien. Ein Verfahren ist die Sterilisation mit Wasserstoffperoxyd.

Aufgabe der Erfindung ist es, ein Verfahren zur Sterilisation von Behältern aus Metall, insbesondere Dosen, zur Verfügung zu stellen, das insbesondere gute Sterilisationsleistungen ermöglicht und gleichzeitig auch in Anlagen mit hohem Durchsatz Anwendung finden kann.

Die Erfindung erreicht dies durch ein Verfahren nach Anspruch 1 sowie einer Vorrichtung nach Anspruch 6.

Ausgestaltungen der Erfindung ergeben sich aus den Unteransprüchen.

Mit dem erfindungsgemäßen Verfahren wird ein flüssiges und/oder dampfförmiges Sterilisationsmittel zunächst in das Innere eines jeden Behälters eingebracht. Vorteilhaft hat sich hierbei die Verwendung von Wasserstoffperoxyd (H₂O₂) als Desinfektionsmittel erwiesen. Dieses wird beispielsweise zunächst feinverstäubt einem Luftstrom, vorteilhafterweise aus steriler Luft, beigemischt und das Gemisch anschließend in einem Verdampfer vollständig verdampft. So entsteht ein Luftstrom, der z.B. bis zur Sättigung mit dampfförmigem H₂O₂ angereichert ist, das in der Folge in das Behälterinnere eingeleitet wird. Dort schlägt es sich auf der im Verhältnis zum Gemisch kälteren Behälterwand nieder und bildet dort einen durchgängigen Flüssigkeitsfilm.

Die Einbringung des Sterilisationsmittels wird beispielsweise mit einer karussellartigen Anordnung erreicht, bei der die Dosen in einer rotierenden Anordnung umlaufen und dabei mit dem Sterilisationsmittel befüllt werden. Im Anschluss werden sie von dem Drehteller wieder entnommen und den Aktivierungseinrichtungen zugeführt.

Hier wird nach dem erfindungsgemäßen Verfahren das Sterilisationsmittel aktiviert und so der Sterilisationsprozess gestartet, was im Falle der vorteilhaften Verwendung von H₂O₂ durch Zuführung einer bestimmten Wärmemenge erfolgt. Bei Erwärmung des niedergeschlagenen H₂O₂ über einen bestimmten Schwellenwert hinaus kommt ein Verfallsprozess in Gang, wobei sich das H₂O₂ zersetzt. Im Zuge des Zersetzungsprozesses entstehen unter anderem freie Radikale in Form atomarer O-und HO-Gruppen, welche mit ggf. vorhandenen Verunreinigungen reagieren und so die eigentliche Sterilisation durchführen, wobei als Endprodukte des Zerfallsprozesses im Wesentlichen Wasser und einige Zerfallsreste übrig bleiben.

Bei der erfindungsgemäßen Ausgestaltung wird der Behälter selbst erwärmt, so dass sich das niedergeschlagene H₂O₂ ebenfalls über den Aktivierungspunkt hinaus erhitzt und der Sterilisationsprozess beginnt. Die Erwärmung des Behälters erfolgt hierbei Induktiv. Das heißt, es wird durch eine geeignete Anordnung, an der der Behälter vorbeigeführt wird, beispielsweise einer Induktionsspule oder dergleichen, durch Erzeugung von Wirbel- oder Ringströmen Im metallischen Behälter selbst eine Erwärmung desselben erreicht.

Zweckmäßig kann dazu eine Reaktionsstrecke verwendet werden, in der die Behälter nach dem Einbringen des Sterilisationsmittels an den induktiven Heizeinrichtungen vorbei transportiert und erwärmt werden. Daran anschließend läuft der Behälter durch eine tunnelförmige Einrichtung, die entgegen der Transportrichtung von einem Luftstrom durchströmt wird, vorzugsweise unter Verwendung von steriler Luft, so dass die bei dem Zerfallsprozess des Sterilisationsmittels entstehenden gasförmigen Zerfallsprodukte aufgenommen und abtransportiert werden können. Am Ende dieser Strecke sind die Behälter sterilisiert und können mit dem Produkt befüllt werden.

In Ausgestaltung ist vorgesehen, dass die zum Verschließen der Behälter vorgesehenen Deckel ebenfalls sterilisiert werden. Dieses erfolgt analog zum beschriebenen Verfahren, indem ein flüssiges oder dampfförmiges Sterilisationsmittel auf die Deckel aufgebracht und anschließend in Ausgestaltung durch Zuführung von Wärme der Sterilisationsprozess in Gang gebracht wird. Hier Ist In Ausgestaltung eine lineare Anordnung vorgesehen, bei dem die Deckel zunächst vereinzelt werden und dann in einer Förderstrecke zunächst mit dem Sterilisationsmittel bedampft bzw. besprüht werden und anschliessend durch Zuführung von Wärme der Zerfallsprozess gestartet wird. Die Anordnung und Dimensionierung der Strecke ist dabei so gewählt, dass der Zerfallsprozess beendet ist und die Deckel im Anschluss unmittelbar dem Verschließer zugeführt werden, indem die befüllten Behälter mit den Deckeln verschlossen werden.

Ggf. kann es zweckmäßig sein, dass die Behälter und/oder die Deckel vor der Befüllung bzw. Verschließung noch einmal mit sterilem Wasser gespült werden.

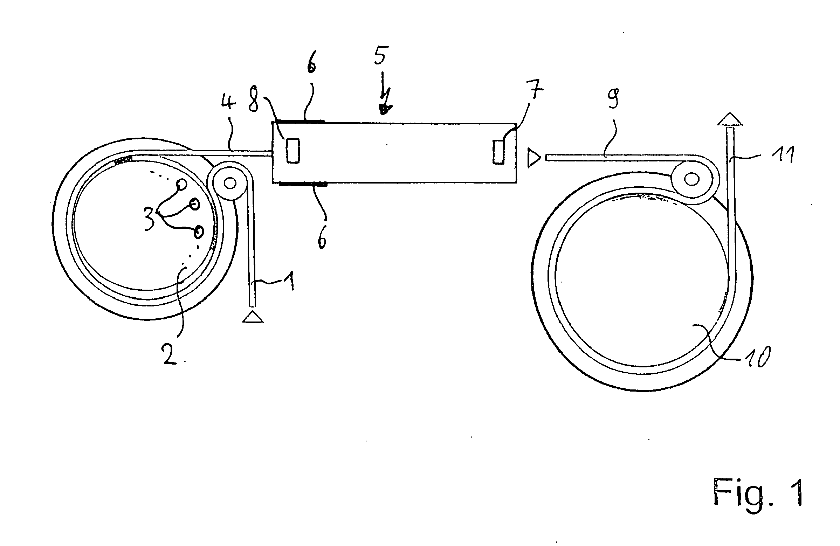

Die Erfindung ist anhand der beigefügten Zeichnung näher erläutert. Diese zeigt in der einzigen Figur eine schematische Draufsicht auf eine Ausgestaltung der Erfindung,

In der Figur ist eine Vorrichtung zur Sterilisation von Behältern aus Metall, insbesondere Dosen für Getränke, in einer ersten Ausgestaltung dargestellt. Sie dient dazu, insbesondere Getränkedosen zu sterilisieren, damit diese in einem aseptischen Füllverfahren befüllt werden können, so dass der Inhalt eine lange Haltbarkeit hat, ohne dass Konservierungsmittel oder Pasteurisationsverfahren und dergleichen verwendet werden müssen.

Dazu werden die leeren metallischen Behälter von einer Fördereinrichtung 1 auf ein Sterilisationskarussell 2 umgesetzt. Dort werden die Behälter im Uhrzeigersinn weiter gedreht. Zusammen mit den Behältem läuft ein Sterilisatorkarussell um, das eine Vielzahl von Sterilisationsköpfen 3 trägt.

Mit den Sterilisationsköpfen 3 wird eine Mischung aus dampfförmigem H₂O₂ und steriler Luft in das Innere der Behälter eingebracht. Dazu wird zunächst flüssiges H₂O₂ fein zerstäubt und einem Strom aus steriler Luft beigemischt. Diese Mischung wird dann durch Verdampferköpfe geführt, die die flüssigen Bestandteile des H₂O₂ verdampfen und so eine gesättigte Mischung aus dampfförmigem H₂O₂ und steriler Luft erzeugen. Diese Mischung weist eine höhere Temperatur als die eingebrachten metallischen Behälter auf, so dass sich das H₂O₂ aus der Mischung auf den kalten Behälterinnenwänden niederschlägt und dort einen gleichmäßigen Flüssigkeitsfilm bildet. Die Dimensionierung des Sterilisatorkarussells ist so ausgelegt, dass bei Beendigung des Umlaufes ein ausreichender Flüssigkeitsfilm im Inneren des Behälters vorhanden ist.

Im Anschluss daran werden die Behälter von einer weiteren Transporteinrichtung 4 einer Reaktionsstrecke 5 zugeführt. Diese ist tunnelförmig ausgebildet. Zu Beginn sind zwei Induktionsspulenanordnungen 6 angebracht, die die vorbeilaufenden metallischen Behälter durch Induzierung von Wirbelströmen erwärmen. Dieses Verfahren findet dann Anwendung, wenn die Dosen, zumindest teilweise, magnetisierbar sind.

Durch die Erwärmung der Dosen wird das darin als gleichmäßiger Film befindliche Sterilisationsmittel über seinen Aktivierungspunkt hinaus erhitzt, worauf bei der Verwendung von H₂O₂ ein Zerfallsprozess beginnt, bei dem über mehrere Zwischenstufen unter anderem freie Radikale wie O- und HO-Gruppen entstehen. Diese reagieren mit möglicherweise vorhandenen Verunreinigungen und führen so den eigentlichen Sterilisationsprozess durch. Am Ende des Prozesses bleibt überwiegend Wasser mit einigen Zerfallsresten übrig, die allerdings biologisch inaktiv sind, so dass die damit behandelten Innenflächen des Behälters im Wesentlichen steril sind.

Nach der erstmaligen Aktivierung durch die Erwärmung der Dosen mit den Induktionsspuleneinrichtungen 6 läuft der Zerfallsprozess selbstständig ab. Die Länge der Reaktionsstrecke 5 ist dabei so bemessen, dass bis zum Ende der Prozess im Wesentlichen abgeschlossen ist. Am Ende des Tunnels befindet sich eine Luftzuführung 7, die sterile Luft in das Innere des Tunnels einleitet. Zu Beginn des Tunnels befindet sich eine Absaugeinrichtung 8, die die Luft aus dem inneren des Tunnels absaugt. So entsteht ein gegen die Laufrichtung der Behälter gerichteter Luftstrom, der etwaig entstehende gasförmige Zerfallsprodukte aufnimmt und aus dem Inneren des Tunnels entfernt.

Am Ende das Tunnels werden die sterilisierten Behälter von einer weiteren Fördereinrichtung 9 dem Füller 10 zugeführt, bei dem die sterilisierten Behälter auf bekannte Art und Weise mit dem einzufüllenden Gut befüllt werden. Anschliessend werden die befüllten Behälter von einer weiteren Transporteinrichtung 11 einem ebenfalls bekannten Verschließer zugeführt.

Dazu ist erfindungsgemäß vorgesehen, dass die zum Verschließen verwendeten Deckel ebenfalls zumindest auf der Innenseite sterilisiert werden.

Dazu ist eine nicht naher dargestellte Deckelsterilisationseinrichtung vorgesehen, die in einer linearen Strecke zunächst, wie bereits beschrieben, einen ausreichend gleichmäßigen Flüssigkeitsfilm aus H₂O₂ auf die Deckel aufbringt und diesen anschließend durch Zuführung von Wärme In Form von erwärmter steriler Luft aktiviert. Nach dem Abschluss des Prozesses werden die so sterilisierten Deckel dann unmittelbar dem Verschließer zugeführt, wo sie auf die gefüllten metallischen Behälter aufgesetzt und diese so verschlossen werden.

Alternativ kann vorgesehen sein, dass sowohl die Behälter als auch die Deckel noch einmal mit sterilem Wasser gespült werden.

Die Erfindung ist nicht auf das vorstehende Ausführungsbeispiel beschränkt, sondern kann in vielfältiger Hinsicht abgewandelt werden, ohne den Grundgedanken zu verlassen.

Die metallischen Behälter müssen nicht zwingend Getränkedosen sein, sondern können auch für andere Anwendungszwecke in der Lebensmittel- oder Getränkeindustrie geeignet sein. Auch Behälter zur Verwendung beispielsweise in der Medizin oder Pharmazie können auf die beschriebene Art und Weise sterilisiert werden. Die Einbringung des Sterilisationsmittels in die Behälter kann auf unterschiedliche Art und Weise geschehen. Neben der Verdampfung mittels Verdampferköpfen sind auch Sprühköpfe oder gar die Zuführung von flüssigen Mitteln ins Innere der Behälter denkbar. Die Ausgestaltung der Anlage Insgesamt ist ebenfalls vielfältigen Variationen zugänglich, so der Konfiguration der einzelnen Anlagenteile oder deren Anordnung.

### Bezugszeichenliste:

- 1: Fördereinrichtung
- 2: Sterilisationskarussell
- 3: Sterilisationsköpfe
- 4: Transporteinrichtung
- 5: Reaktionsstrecke
- 6: Induktionsspulenanordnungen
- 7: Luftzuführung
- 8: Absaugeinrichtung
- 9: Fördereinrichtung
- 10: Füller
- 11: Transporteinrichtung

## Patentansprüche

1. Verfahren zur Sterilisation von Behältern aus Metall, insbesondere Dosen, sowie der zum Verschließen der Behälter vorgesehenen Deckel, wobei ein flüssiges und/oder dampfförmiges Sterilisationsmittel In das Innere der Behälter eingebracht wird, und dass anschließend das Sterilisationsmittel zur Aktivierung erwärmt und so der Sterilisationsprozess gestartet wird, **dadurch gekennzeichnet, dass** das Sterilisationsmittel durch eine Aufheizung der Behälter und/oder der Deckel aktiviert wird, wobei die Behälter Induktiv aufgeheizt werden.

2. Verfahren nach Anspruch 1, **dadurch gekennzeichnet, dass** das flüssige und/oder dampfförmige Sterilisationsmittel auf die Deckel aufgebracht wird, und dass anschließen das Sterilisationsmittel aktiviert und so der Sterilisationsprozess gestartet wird.

3. Verfahren nach Anspruch 1 oder einem der folgenden, **dadurch gekennzeichnet, dass** das Einbringen in den Behälter und/oder das Aufbringen auf die Deckel durch mit dem flüssigen und/oder dampfförmigen Sterilisationsmittel gesättigte Luft, insbesondere sterile Luft, erfolgt.

4. Verfahren nach Anspruch 1 oder einem der folgenden, **dadurch gekennzeichnet, dass** als flüssiges und/oder dampfförmiges Sterilisationsmittel, flüssiges und/oder dampfförmiges Wasserstoffperoxyd (H₂O₂) verwendet wird.

5. Verfahren nach Anspruch 1 oder einem der folgenden, **dadurch gekennzeichnet, dass** zum Abtransport der beim Sterilisationsprozess entstehenden Zertallsprodukte und/oder Sterilisationsmittelreste Luft, Insbesondere sterile Luft, verwendet wird.

6. Vorrichtung zur Sterilisation von Behältern aus Metall, insbesondere Dosen, wobei eine Einrichtung zur Einbringung eines flüssigen und/oder dampfförmigen Sterilisationsmittels in das Innere der Behälter und eine Einrichtung zur Aktivierung des Sterilisationsmittels vorgesehen sind, **dadurch gekennzeichnet, dass** zur Aktivierung des Sterilisationsmittels wenigstens eine Induktionsspuleneinrichtung (6) zur induktiven Erwärmung der Behälter vorgesehen ist.

7. Vorrichtung nach Anspruch 6, **dadurch gekennzeichnet, dass** eine zusätzliche Sterilisationsvorrichtung für die zum Verschließen der Behälter vorgesehenen Deckel vorgesehen ist.

8. Vorrichtung nach Anspruch 6 oder 7, **dadurch gekennzeichnet, dass** als Einrichtung zur Einbringung eines flüssigen und/oder dampfförmigen Sterilisationsmittels in das Innere der Behälter eine Verteileinrichtung für eine Mischung aus flüssigem und/oder dampfförmigem Wasserstoffperoxyd (H₂O₂) und Luft vorgesehen ist.

9. Vorrichtung nach Anspruch 6, **dadurch gekennzeichnet, dass** eine Fördereinrichtung zum Transport der zu sterilisierenden Behälter an der wenigstens einen Induktionsspuleneinrichtung (6) vorbei vorgesehen ist.

10. Vorrichtung nach Anspruch 9, **dadurch gekennzeichnet, dass** im Anschluss an die wenigstens eine Induktionsspuleneinrichtung (6) eine Reaktionsstrecke (5) zum Ablauf des Sterilisationsprozesses vorgesehen ist und ein gegen die Förderrichtung laufender Luftstrom, Insbesondere aus steriler Luft, vorgesehen ist.

11. Vorrichtung nach Anspruch 6, **dadurch gekennzeichnet, dass** eine Vorrichtung zur Einbringung eines durch Erwärmung aktivierbaren Sterilisationsmittels, ins Innere der Behälter vorgesehen ist.

## Claims

1. Method for sterilising metal containers, in particular cans, and the lids provided for closing the containers, wherein a liquid and/or vaporous sterilisation agent is introduced into the interior of the containers, and that then the sterilisation agent is heated for activation and thus the sterilisation process is started, **characterised in that** the sterilisation agent is activated by heating the containers and/or lids, wherein the containers are inductively heated.

2. Method according to claim 1, **characterised in that** the liquid and/or vaporous sterilisation agent is applied to the lid, and then the sterilisation agent is activated and thus the sterilisation process is started.

3. Method according to claim 1 or one of the preceding claims, **characterised in that** introduction into the container and/or application onto the lid takes place by means of air, in particular sterile air, saturated with the liquid and/or vaporous sterilisation agent.

4. Method according to claim 1 or one of the preceding claims, **characterised in that** liquid and/or vaporous hydrogen peroxide (H₂O₂) is used as the liquid and/or vaporous sterilisation agent.

5. Method according to claim 1 or one of the preceding claims, **characterised in that** air, in particular sterile air, is used to discharge the waste products occurring during the sterilisation process and/or the sterilisation agent residue.

6. Device for sterilising metal containers, in particular cans, wherein a device for introducing a liquid and/or vaporous sterilisation agent into the interior of the container and a device for activating the sterilisation agent are provided, **characterised in that** to activate the sterilisation agent, at least one induction coil device (6) is provided for inductive heating of the containers.

7. Device according to claim 6, **characterised in that** an additional sterilisation device is provided for the lids intended for closing the containers.

8. Device according to claim 6 or 7, **characterised in that** a distribution device for a mixture of liquid and/or vaporous hydrogen peroxide (H₂O₂) and air is provided as a device for introducing a liquid and/or vaporous sterilisation agent into the interior of the containers.

9. Device according to claim 6, **characterised in that** a delivery device is provided for transporting the containers to be sterilised past the at least one induction coil device (6).

10. Device according to claim 9, **characterised in that** connected to the at least one induction coil device (6), a reaction line (5) for performance of the sterilisation process is provided, and an air flow, in particular of sterile air, directed against the delivery device is provided.

11. Device according to claim 6, **characterised in that** a device is provided for introducing a sterilisation agent which can be activated by heating into the interior of the containers.

## Revendications

1. Procédé de stérilisation de contenants en métal, en particulier, de boîtes, et de couvercles prévus pour fermer le contenant, dans lequel un agent de stérilisation liquide et/ou sous forme de vapeur est inséré à l'intérieur du contenant, et ensuite, l'agent de stérilisation est chauffé pour être activé et ainsi, le processus de stérilisation est lancé, **caractérisé en ce que** l'agent de stérilisation est activé par un chauffage du contenant et/ou du couvercle, le contenant étant chauffé par induction.

2. Procédé selon la revendication 1, **caractérisé en ce que** l'agent de stérilisation liquide et/ou sous forme de vapeur est appliqué sur le couvercle, et **en ce qu'**ensuite, l'agent de stérilisation est activé et ainsi, le processus de stérilisation est lancé.

3. Procédé selon la revendication 1 ou l'une des suivantes, **caractérisé en ce que** l'insertion dans le contenant et/ou l'application sur le couvercle s'effectue par de l'air saturé avec l'agent de stérilisation liquide et/ou sous forme de vapeur, en particulier, de l'air stérile.

4. Procédé selon la revendication 1 ou l'une des suivantes, **caractérisé en ce que** l'on utilise comme agent de stérilisation liquide et/ou sous forme de vapeur, du peroxyde d'hydrogène (H₂O₂) liquide et/ou sous forme de vapeur.

5. Procédé selon la revendication 1 ou l'une des suivantes, **caractérisé en ce que**, pour l'évacuation des produits de décomposition et/ou des résidus de stérilisation obtenus lors du processus de stérilisation, on utilise, en particulier, de l'air stérile.

6. Dispositif de stérilisation de contenants en métal, en particulier, de boîtes, un dispositif d'insertion d'un agent de stérilisation liquide et/ou sous forme de vapeur étant prévu à l'intérieur du contenant et un système d'activation de l'agent de stérilisation étant prévu, **caractérisé en ce que**, pour l'activation de l'agent de stérilisation, au moins un système de bobine d'induction (6) est prévu pour le chauffage par induction du contenant.

7. Dispositif selon la revendication 6, **caractérisé en ce qu'**un dispositif de stérilisation supplémentaire est prévu pour le couvercle prévu pour la fermeture du contenant.

8. Dispositif selon la revendication 6 ou 7, **caractérisé en ce qu'**est prévu comme système d'apport d'un agent de stérilisation liquide et/ou sous forme de vapeur à l'intérieur du contenant, un système de répartition d'un mélange de peroxyde d'hydrogène (H₂O₂) liquide et/ou sous forme de vapeur et d'air.

9. Dispositif selon la revendication 6, **caractérisé en ce qu'**un dispositif d'acheminement est prévu pour le transport du contenant à stériliser sur l'au moins un système de bobine à induction (6).

10. Dispositif selon la revendication 9, **caractérisé en ce qu'**à la suite de l'au moins un système de bobine à induction (6) est prévu un trajet réactionnel (5) pour le déroulement du processus de stérilisation et un courant d'air allant à contre-courant du dispositif d'acheminement, en particulier d'air stérile, est prévu.

11. Dispositif selon la revendication 6, **caractérisé en ce qu'**un dispositif d'insertion d'un agent de stérilisation activable par chauffage est prévu, à l'intérieur du contenant.
